# EUROPEAN PATENT APPLICATION

(11) **EP 2 730 283 A1**
(43) Date of publication of application: **14.05.2014**
(21) Application number: 12802941.0
(22) Date of filing: 20.06.2012
(51) Int. Cl.: A61K 31/5575, A61P 17/00, A61P 17/14, A61P 29/00, A61P 37/02, A61P 37/06, A61P 37/08, C07C 69/734, C07D 317/30, C07D 319/06

(54) **PHARMACEUTICAL COMPOSITION FOR INFLAMMATORY DISEASES, ALLERGIC DISEASES AND AUTOIMMUNE DISEASES**

(30) Priority: 21.06.2011 JP 2011137213
(71) Applicant: R-Tech Ueno, Ltd., Tokyo 100-0011 (JP)
(72) Inventor: MASHIMA, Yukihiko, Tokyo 100-0011 (JP); TAJIMA, Masahiro, Tokyo 100-0011 (JP); TABUCHI, Reiko, Tokyo 100-0011 (JP)
(74) Representative: Albrecht, Thomas
(86) International application number: PCT/JP2012/065706
(87) International publication number: WO 2012/176792

(57) **Abstract**

The present invention relates to methods and pharmaceutical compositions for immunosuppression by using the fatty acid derivative represented by formula (I). The present invention further relates to methods and pharmaceutical compositions for treating inflammatory diseases, allergic diseases and autoimmune diseases by using said fatty acid derivative.

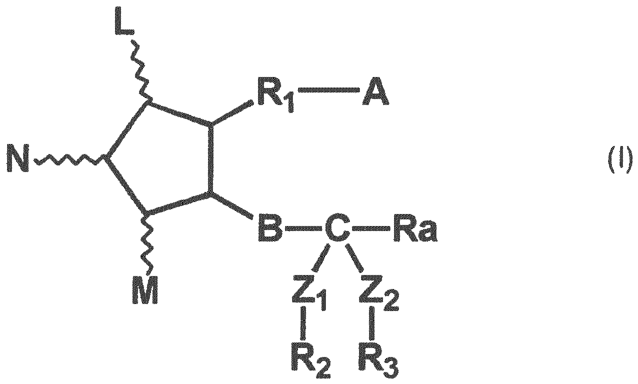

## Description

### TECHNICAL FIELD

The present invention relates to methods and pharmaceutical compositions for immunosuppression by a fatty acid derivative having a specific structure. Further the present invention relates to methods and pharmaceutical compositions for the treatment of inflammatory diseases, allergic diseases, and autoimmune diseases.

### BACKGROUND ART

Cells involved in adaptive immunity belong to a type of white blood cell, which is called as lymphocyte. Lymphocytes are roughly divided into B cells and T cells, both derived from hematopoietic stem cells in the bone marrow, and B cells mature independently of the thymus while T cells differentiate and mature in the thymus. Both B cells and T cells recognize specific target antigens, and B cells are involved in the humoral immune response while T cells are involved in cell-mediated immune response. Hematopoietic stem cells in the bone marrow differentiate into pre-B cells and mature B cells upon stimulation with cytokines such as interleukin or the like. The mature B cells are activated in response to antigen stimulation, and finally become plasma cells having antibody-producing ability. Also, hematopoietic stem cells in the bone marrow differentiate into pre-T cells, and the pre-T cells move to the thymus and repeat differentiation to become mature T cells. Then, the mature T cells are activated by antigen stimulation and become activated T cells having proliferation ability or cytotoxic activity. Therefore, it is possible to suppress immune response by inhibiting the proliferation of the activated B cells and T cells.

Inflammation is the first response of the immune system caused after infection or injury. Whereas transient inflammation is effective for protection from infection and injury, uncontrolled inflammation causes damage on the tissue and becomes a potential cause of many diseases. In general, inflammation is caused by antigen binding to the T cell antigen receptor.

An immune response to foreign antigens such as bacteria and viruses allows for defending against and eliminating infection therefrom. However, an abnormal immune response that is not controlled causes autoimmune diseases and allergic diseases. Allergic disease is a disease in which the immune response against foreign antigens occurs excessively and it is characterized by T cell activation and overproduction of IgE by B cells. On the other hand, autoimmune disease is a disease in which the immune response against autologous antigens occurs and autoantibodies cause various disorders. Autoimmune disease is characterized by activation of T cells and B cells and overproduction of inflammatory cytokines.

Allergic diseases and autoimmune diseases are closely related to each other because many of them are caused by inflammation. Also, many diseases considered as inflammatory diseases overlap with autoimmune diseases and allergic diseases.

Fatty acid derivatives, members of class of organic carboxylic acids, which are contained in tissues or organs of human or other mammals, and exhibit a wide range of physiological activity. Some of naturally occurring fatty acid derivatives generally have a prostanoic acid skeleton as shown in the formula (A):

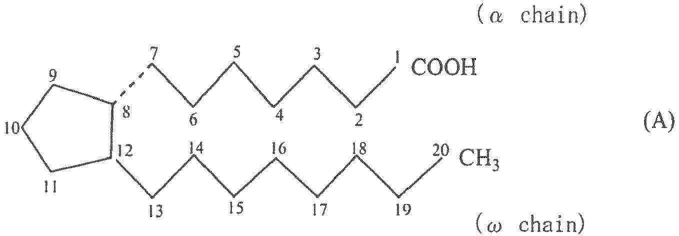

On the other hand, some of synthetic analogues of naturally occurring prostaglandins (hereinafter, referred to as PG(s)) have modified skeletons. The naturally occurring PGs are classified into PGAs, PGBs, PGCs, PGDs, PGEs, PGFs, PGGs, PGHs, PGIs and PGJs according to the structure of the five-membered ring moiety thereof and further classified into the following three types according to the number and position of the unsaturated bond at the carbon chain moiety thereof:
Subscript 1: 13,14-unsaturated-15-OH
Subscript 2: 5,6- and 13,14-diunsaturated-15-OH
Subscript 3: 5,6-, 13,14-,and 17,18-triunsaturated-15-OH.

Further, the PGFs are classified, according to the configuration of the hydroxyl groups at positions 9 and 11, into α type (the hydroxyl group is of an α-configuration) and β type (the hydroxyl group is of a β-configuration).

Certain fatty acid derivatives having two hetero atoms at position 15 are known in the art. U.S. Patent No. 4088775 (Patent Literature 1) discloses specific 15-ethylenedioxy-fatty acid derivatives. U.S. Patent No. 4870104 (Patent Literature 2) discloses 11-halogen-fatty acid derivatives which may have an ethylenedioxy methylene group at position 15 and its use as agents inhibiting gastric acid secretion. U.S. Patent No. 6353014 (Patent Literature 3) discloses specific 15-ketal fatty acid derivatives having hydroxy groups at positions 9 and 11 being useful for the treatment of glaucoma and ocular hypertension. WO2005/01392 (Patent Literature 4) and WO2006/070942(Patent Literature 5) disclose that fatty acid derivatives having two hetero atoms on the carbon atom at position 15 are useful for promoting hair growth.

Those related art references neither disclose nor suggest that fatty acid derivatives having two hetero atoms on the carbon atom at position 15 suppress proliferation of activated B cells and/or T cells and would be useful to treat inflammatory diseases, allergic diseases, and autoimmune diseases.

### CITATION LIST

### PATENT DOCUMENTS

Patent Document 1: U.S. Patent No.4088775
Patent Document 2: U.S. Patent No.4870104
Patent Document 3: U.S. Patent No.6353014
Patent Document 4: WO2005/013928
Patent Document 5: WO2006/070942

### (Those references are herein incorporated by reference.)

### SUMMARY OF THE INVENTION

An object of the present invention is to provide methods and pharmaceutical compositions for immunosuppression, and further methods and pharmaceutical compositions for the treatment of inflammatory diseases, allergic diseases, and autoimmune diseases.

The inventors have found that fatty acid derivatives having a specific structure can suppress immune response by suppressing proliferation of activated B cells and/or T cells, and thus the fatty acid derivatives areuseful to treat inflammatory diseases, allergic diseases, and autoimmune diseases.

The present invention relates to followings.
(1) A pharmaceutical composition for immunosuppression which comprises as an active ingredient a fatty acid derivative represented by formula (I): 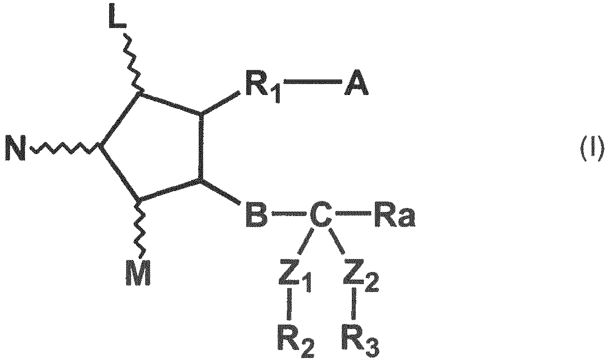
   wherein L, M and N are hydrogen, hydroxy, halogen, lower alkyl, hydroxy(lower)alkyl, lower alkanoyloxy or oxo, wherein at least one of L and M is a group other than hydrogen, and the five-membered ring may have at least one double bond;
   A is -CH₃, or -CH₂OH, -COCH₂OH, -COOH or a functional derivative thereof;
   B is a single bond, -CH₂-CH₂-, -CH=CH-, -C≡C-,-CH₂-CH₂-CH₂-, -CH=CH-CH₂-, -CH₂-CH=CH-, -C≡C-CH₂- or -CH₂-C≡C- ;
   Z₁ and Z₂ are oxygen, nitrogen or sulfur,
   R₂ and R₃ are lower alkyl, or R₂ and R₃ are optionally linked together to form lower alkylene,
   R₁ is a saturated or unsaturated bivalent lower or medium aliphatic hydrocarbon residue, which is unsubstituted or substituted with halogen, alkyl, hydroxy, oxo, aryl or heterocyclic group, and at least one of carbon atoms in the aliphatic hydrocarbon is optionally replaced by oxygen, nitrogen or sulfur; and
   Ra is a saturated or unsaturated lower or medium aliphatic hydrocarbon residue, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.
(2) The pharmaceutical composition according to (1), wherein L and M are hydroxy.
(3) The pharmaceutical composition according to (1) or (2), wherein A is -COOH or a functional derivative thereof.
(4) The pharmaceutical composition according to any one of (1)-(3), wherein B is -CH₂-CH₂-, -CH=CH-, or -C≡C-.
(5) The pharmaceutical composition according to any one of (1)-(4), wherein Z₁ and Z₂ are oxygen.
(6) The pharmaceutical composition according to any one of (1)-(5), wherein R₂ and R₃ are linked together to form lower alkylene,
(7) The pharmaceutical composition according to (6), wherein the lower alkylene is C3 alkylene.
(8) The pharmaceutical composition according to any one of (1)-(7), wherein the fatty acid derivative is isopropyl (Z)-7-[(1R,2R,3R,5S)-2-(3,3-ethylenedioxydecyl)-3,5-dihydroxycyclopentyl]hept-5-enoate.
(9) The pharmaceutical composition according to any one of (1)-(8) for the treatment of an inflammatory disease.
(10) The pharmaceutical composition according to (9) for treating skin inflammation or alopecia associated therewith.
(11) The pharmaceutical composition according to (10) for treating alopecia associated with skin inflammation.
(12) The pharmaceutical composition according to any one of (1)-(8) for the treatment of an allergic disease.
(13) The pharmaceutical composition according to (12) for treating atopic dermatitis or alopecia associated therewith.
(14) The pharmaceutical composition according to (13) for treating alopecia associated with atopic dermatitis.
(15) The pharmaceutical composition according to any one of (1)-(8) for the treatment of an autoimmune disease.
(16) A method for immunosuppression in a mammalian subject, which comprises administering an effective amount of the fatty acid derivative of formula (I) as defined in (1) to the subject in need thereof.
(17) Use of the fatty acid derivative of formula (I) as defined in (1) for immunosuppression in a mammalian subject.

### BRIEF DESCRIPTION OF DRAWINGS

[Fig 1] Results of B cell proliferation suppression test of compound A.
[Fig 2] Results of T cell proliferation suppression test of compound A.
[Fig 3] Results of NF-AT gene transcription suppression test of compound A.
[Fig 4] Results of NF-κB gene transcription suppression test of compound A.

### DESCRIPTION OF EMBODIMENTS

The nomenclature of fatty acid derivatives used herein is based on the numbering system of prostanoic acid represented in the above formula (A).

The formula (A) shows a basic skeleton of the C-20 compound, but the present invention is not limited to those having the same number of carbon atoms. In the formula (A), the numbering of the carbon atoms which constitute the basic skeleton of the compounds starts at the carboxylic acid (numbered 1), and carbon atoms in the α-chain are numbered 2 to 7 towards the five-membered ring, those in the ring are 8 to 12, and those in the ω-chain are 13 to 20. When the number of carbon atoms is decreased in the α-chain, the number is deleted in the order starting from position 2; and when the number of carbon atoms is increased in the α-chain, compounds are named as substitution compounds having respective substituents at position 2 in place of carboxy group (C-1). Similarly, when the number of carbon atoms is decreased in the ω-chain, the number is deleted in the order starting from position 20; and when the number of carbon atoms is increased in the ω-chain, the carbon atoms at the position 21 or later are named as a substituent at position 20. Stereochemistry of the compounds is the same as that of the above formula (A) unless otherwise specified.

In general, each of PGD, PGE and PGF represents a fatty acid derivative having hydroxy groups at positions 9 and/or 11, but in the present specification they also include those having substituents other than the hydroxy groups at positions 9 and/or 11. Such compounds are referred to as 9-deoxy-9-substituted compounds or 11-deoxy-11-substituted compounds. A compound having hydrogen in place of the hydroxy group is simply named as 9- or 11-deoxy compound.

As stated above, the nomenclature of fatty acid derivatives is based on the prostanoic acid skeleton. In the case the compound has similar partial structure as the naturally occurring fatty acid derivative, the abbreviation of "PG" may be used. Thus, a PG compound whose α-chain is extended by two carbon atoms, that is, a fatty acid derivative having 9 carbon atoms in the α-chain may be named as 2-decarboxy-2-(2-carboxyethyl)-PG compound. Similarly, a fatty acid derivative having 11 carbon atoms in the α-chain may be named as 2-decarboxy-2-(4-carboxybutyl)-PG compound. Further, a fatty acid derivative whose ω-chain is extended by two carbon atoms, that is, having 10 carbon atoms in the ω-chain may be named as 20-ethyl-PG compound. These compounds, however, may also be named according to the IUPAC nomenclatures.

The fatty acid derivative used in the present invention may be any substitution compound or derivative of a fatty acid derivative. The fatty acid derivatives may include a fatty acid derivative having one double bond between positions 13 and 14, and a hydroxy group at position 15; a fatty acid derivative having one additional double bound between positions 5 and 6; and a fatty acid derivative having a further double bond between positions 17 and 18; a fatty acid derivative having oxo group at position 15 instead of the hydroxy group; a fatty acid derivative having hydrogen instead of the hydroxy group at position 15; a fatty acid derivative having fluorine at position 15 instead of the hydroxy group; and a derivative of the aforesaid derivative having a single or triple bond instead of the double bond between positions 13 and 14. Furthermore, examples of the analogues of the fatty acid derivative including substitution compounds or derivatives include a compound whose carboxy group at the end of the α chain is esterified or amidated, or a physiologically acceptable salt thereof; a compound whose α or ω chain is shortened or extended than that of the naturally occurring PG; a compound having a side chain having, for example, 1-3 carbon atoms, on their α or ω chain; a compound having a substituent such as hydroxy, halogen, lower alkyl, hydroxy(lower)alkyl or oxo, or a double bond on its five membered ring; a compound having a substituent such as halogen, oxo, aryl and heterocyclic group on its α chain; a compound having a substituent such as halogen, oxo, hydroxy, lower alkoxy, lower alkanoyloxy, cyclo(lower)alkyl, cyclo(lower)alkyloxy, aryl, aryloxy, heterocyclic or heterocyclic-oxy on its ω chain; and a compound having shorter ω chain than that of normal prostanoic acid and having a substituent such as lower alkoxy, lower alkanoyloxy, cyclo(lower)alkyl, cyclo(lower)alkyloxy, aryl, aryloxy, heterocyclic or heterocyclic-oxy group at the end of the ω chain.

A preferred prostaglandin compound used in the present invention is represented by formula (I): 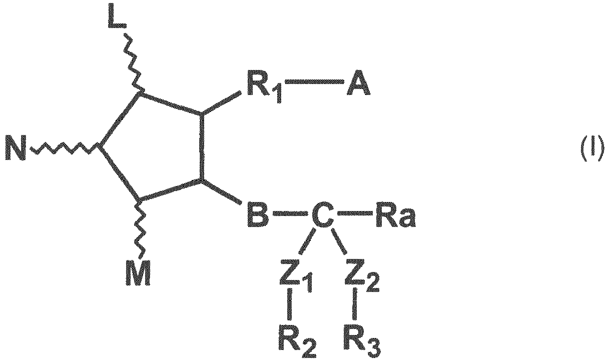
wherein L, M and N are hydrogen, hydroxy, halogen, lower alkyl, hydroxy(lower)alkyl, lower alkanoyloxy or oxo, wherein at least one of L and M is a group other than hydrogen, and the five-membered ring may have at least one double bond;
A is -CH₃, or -CH₂OH, -COCH₂OH, -COOH or a functional derivative thereof;
B is a single bond, -CH₂-CH₂-, -CH=CH-, -C≡C-,-CH₂-CH₂-CH₂, -CH=CH-CH₂-, -CH₂-CH=CH-, -C≡C-CH₂- or -CH₂-C≡C-;
Z₁ and Z₂ are oxygen, nitrogen or sulfur,
R₂ and R₃ are lower alkyl, or R₂ and R₃ are optionally linked together to form lower alkylene,
R₁ is a saturated or unsaturated bivalent lower or medium aliphatic hydrocarbon residue, which is unsubstituted or substituted with halogen, alkyl, hydroxy, oxo, aryl or heterocyclic group, and at least one of carbon atoms in the aliphatic hydrocarbon is optionally replaced by oxygen, nitrogen or sulfur; and
Ra is a saturated or unsaturated lower or medium aliphatic hydrocarbon residue, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.

A preferred fatty acid derivative used in the present invention is represented by the formula (II): 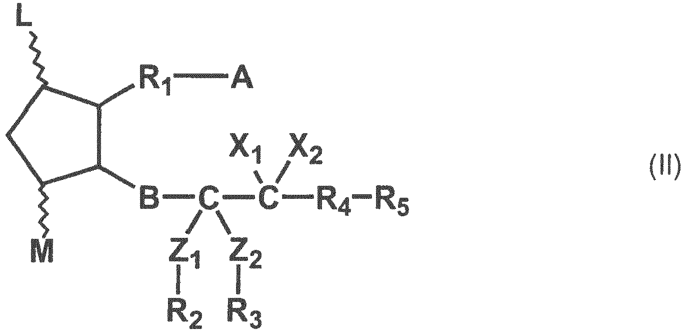
wherein L and M are hydrogen, hydroxy, halogen, lower alkyl, hydroxy(lower)alkyl, lower alkanoyloxy or oxo, wherein at least one of L and M is a group other than hydrogen, and the five-membered ring may have one or more double bonds;
A is -CH₃, or -CH₂OH, -COCH₂OH, -COOH or a functional derivative thereof;
B is a single bond, -CH₂-CH₂-, -CH=CH-, -C≡C-, - CH₂-CH₂-CH₂-, -CH=CH-CH₂-, -CH₂-CH=CH-, -C≡C-CH₂- or -CH₂-C≡C-;
Z₁ and Z₂ are oxygen, nitrogen or sulfur,
R₂ and R₃ are lower alkyl, or R₂ and R₃ are optionally linked together to form lower alkylene,
X₁ and X₂ are hydrogen, lower alkyl, or halogen;
R₁ is a saturated or unsaturated bivalent lower or medium aliphatic hydrocarbon residue, which is unsubstituted or substituted with halogen, alkyl, hydroxy, oxo, aryl or heterocyclic group, and at least one of carbon atoms in the aliphatic hydrocarbon is optionally replaced by oxygen, nitrogen or sulfur;
R₄ is a single bond or lower alkylene; and
R₅ is lower alkyl, lower alkoxy, lower alkanoyloxy, cyclo(lower)alkyl, cyclo(lower)alkyloxy, aryl, aryloxy, heterocyclic group or heterocyclic-oxy group.

In the above formula, the term "unsaturated" in the definitions for R₁ and Ra is intended to include at least one or more double bonds and/or triple bonds that are isolatedly, separately or serially present between carbon atoms of the main and/or side chains. According to the usual nomenclature, an unsaturated bond between two serial positions is represented by denoting the lower number of the two positions, and an unsaturated bond between two distal positions is represented by denoting both of the positions.

The term "lower or medium aliphatic hydrocarbon" refers to a straight or branched chain hydrocarbon group having 1 to 14 carbon atoms (for a side chain, 1 to 3 carbon atoms are preferable) and preferably 1 to 10, especially 1 to 8 carbon atoms.

The term "halogen atom" covers fluorine, chlorine, bromine and iodine.

The term "lower" throughout the specification is intended to include a group having 1 to 6 carbon atoms unless otherwise specified.

The term "lower alkyl" refers to a straight or branched chain saturated hydrocarbon group containing 1 to 6 carbon atoms and includes, for example, methyl, ethyl, propyl, isopropyl, butyl, isobutyl, t-butyl, pentyl and hexyl.

The term "lower alkylene" refers to a straight or branched chain bivalent saturated hydrocarbon group containing 1 to 6 carbon atoms and includes, for example, methylene, ethylene, propylene, isopropylene, butylene, isobutylene, t-butylene, pentylene and hexylene.

The term "lower alkoxy" refers to a group of lower alkyl-O-, wherein lower alkyl is as defined above.

The term "hydroxy (lower) alkyl" refers to a lower alkyl as defined above which is substituted with at least one hydroxy group such as hydroxymethyl, 1-hydroxyethyl, 2-hydroxyethyl and 1-methyl-1-hydroxyethyl.

The term "lower alkanoyloxy" refers to a group represented by the formula RCO-O-, wherein RCO- is an acyl group formed by oxidation of a lower alkyl group as defined above such as acetyl.

The term "cyclo(lower)alkyl" refers to a cyclic group formed by cyclization of a lower alkyl group as defined above but contains three or more carbon atoms, and includes, for example, cyclopropyl, cyclobutyl, cyclopentyl and cyclohexyl.

The term "cyclo(lower)alkyloxy" refers to the group of cyclo(lower)alkyl-O-, wherein cyclo(lower)alkyl is as defined above.

The term "aryl" may include unsubstituted or substituted aromatic hydrocarbon rings (preferably monocyclic groups), for example, phenyl, tolyl, and xylyl. Examples of the substituents are halogen atom and halo(lower)alkyl, wherein halogen atom and lower alkyl are as defined above.

The term "aryloxy" refers to a group represented by the formula ArO-, wherein Ar is aryl as defined above.

The term "heterocyclic group" may include monoto tri-cyclic, preferably monocyclic heterocyclic group which is 5 to 14, preferably 5 to 10 membered ring having optionally substituted carbon atoms and 1 to 4, preferably 1 to 3 of 1 or 2 types of hetero atoms selected from nitrogen atom, oxygen atom and sulfur atom. Examples of the heterocyclic group include furyl, thienyl, pyrrolyl, oxazolyl, isoxazolyl, thiazolyl, isothiazolyl, imidazolyl, pyrazolyl, furazanyl, pyranyl, pyridyl, pyridazinyl, pyrimidyl, pyrazinyl, 2-pyrrolinyl, pyrrolidinyl, 2-imidazolinyl, imidazolidinyl, 2-pyrazolinyl, pyrazolidinyl, piperidino, piperazinyl, morpholino, indolyl, benzothienyl, quinolyl, isoquinolyl, purinyl, quinazolinyl, carbazolyl, acridinyl, phenanthridinyl, benzimidazolyl, benzimidazolinyl, benzothiazolyl, phenothiazinyl. Examples of the substituent in this case include halogen, and halogen substituted lower alkyl group, wherein halogen atom and lower alkyl group are as described above.

The term "heterocyclic-oxy group" means a group represented by the formula HcO-, wherein Hc is a heterocyclic group as described above.

The term "functional derivative" of A includes salts (preferably pharmaceutically acceptable salts), ethers, esters and amides.

Suitable "pharmaceutically acceptable salts" include conventionally used non-toxic salts, for example an inorganic base salt such as an alkali metal salt (such as sodium salt and potassium salt), an alkaline earth metal salt (such as calcium salt and magnesium salt), an ammonium salt; or an organic base salt, for example, an amine salt (such as methylamine salt, dimethylamine salt, cyclohexylamine salt, benzylamine salt, piperidine salt, ethylenediamine salt, ethanolamine salt, diethanolamine salt, triethanolamine salt, tris(hydroxymethylamino)ethane salt, monomethyl- monoethanolamine salt, procaine salt and caffeine salt), a basic amino acid salt (such as arginine salt and lysine salt), tetraalkyl ammonium salt and the like. These salts may be prepared by a conventional reaction between the corresponding acid and base, or by salt interchange.

Examples of the ethers include alkyl ethers, for example, lower alkyl ethers such as methyl ether, ethyl ether, propyl ether, isopropyl ether, butyl ether, isobutyl ether, t-butyl ether, pentyl ether and 1-cyclopropyl ethyl ether; and medium or higher alkyl ethers such as octyl ether, diethylhexyl ether, lauryl ether and cetyl ether; unsaturated ethers such as oleyl ether and linolenyl ether; lower alkenyl ethers such as vinyl ether, and allyl ether; lower alkynyl ethers such as ethynyl ether and propynyl ether; hydroxy(lower)alkyl ethers such as hydroxyethyl ether and hydroxyisopropyl ether; lower alkoxy (lower)alkyl ethers such as methoxymethyl ether and 1-methoxyethyl ether; optionally substituted aryl ethers such as phenyl ether, tosyl ether, t-butylphenyl ether, salicyl ether, 3,4-di-methoxyphenyl ether and benzamidophenyl ether; and aryl(lower)alkyl ethers such as benzyl ether, trityl ether and benzhydryl ether.

Examples of the esters include aliphatic esters, for example, lower alkyl esters such as methyl ester, ethyl ester, propyl ester, isopropyl ester, butyl ester, isobutyl ester, t-butyl ester, pentyl ester and 1-cyclopropylethyl ester; lower alkenyl esters such as vinyl ester and allyl ester; lower alkynyl esters such as ethynyl ester and propynyl ester; hydroxy(lower)alkyl ester such as hydroxyethyl ester; lower alkoxy (lower) alkyl esters such as methoxymethyl ester and 1-methoxyethyl ester; and optionally substituted aryl esters such as, for example, phenyl ester, tolyl ester, t-butylphenyl ester, salicyl ester, 3,4-di-methoxyphenyl ester and benzamidophenyl ester; and aryl(lower)alkyl ester such as benzyl ester, trityl ester and benzhydryl ester.

The amide of A mean a group represented by the formula -CONR'R", wherein each of R' and R" is hydrogen, lower alkyl, aryl, alkyl- or aryl-sulfonyl, lower alkenyl and lower alkynyl, and include for example lower alkyl amides such as methylamide, ethylamide, dimethylamide and diethylamide; arylamides such as anilide and toluidide; and alkyl- or aryl-sulfonylamides such as methylsulfonylamide, ethylsulfonyl-amide and tolylsulfonylamide.

Preferred examples of L and M include hydrogen, hydroxy and oxo, and preferablly, L is hydroxy and M is hydroxy.

Preferred example of A is -COOH or a functional derivative thereof.

Preferred example of B is -CH₂-CH₂-, -CH=CH- or - C≡C-.

Preferred example of Z₁ and Z₂ is both oxygen.

Preferred examples of R₂ and R₃ are those linked together to form lower alkylene, preferably C3 alkylene.

Preferred example of X₁ and X₂ is hydrogen or halogen, and more preferably, both X₁ and X₂ are hydrogen or fluorine.

Preferred R₁ is a hydrocarbon residue containing 1-10 carbon atoms, preferably 6-10 carbon atoms. Further, at least one carbon atom in the aliphatic hydrocarbon is optionally replaced by oxygen, nitrogen or sulfur.

Examples of R₁ include, for example, the following groups:
-CH₂-CH₂-CH₂-CH₂-CH₂-CH₂-,
-CH₂-CH=CH-CH₂-CH₂-CH₂-,
-CH₂-CH₂-CH₂-CH₂-CH=CH-,
-CH₂-C≡C-CH₂-CH₂-CH₂-,
-CH₂-CH₂-CH₂-CH₂-O-CH₂-,
-CH₂-CH=CH-CH₂-O-CH₂-,
-CH₂-C≡C-CH₂-O-CH₂-,
-CH₂-CH₂-CH₂-CH₂-CH₂-CH₂-CH₂-,
-CH₂-CH=CH-CH₂-CH₂-CH₂-CH₂-,
-CH₂-CH₂-CH₂-CH₂-CH₂-CH=CH-,
-CH₂-C≡C-CH₂-CH₂-CH₂-CH₂-,
-CH₂-CH₂-CH₂-CH₂-CH₂-CH(CH₃) -CH₂-,
-CH₂-CH₂-CH₂-CH₂-CH(CH₃) -CH₂-,
-CH₂-CH₂-CH₂-CH₂-CH₂-CH₂-CH₂-CH₂-,
-CH₂-CH=CH-CH₂-CH₂-CH₂-CH₂-CH₂-,
-CH₂-CH₂-CH₂-CH₂-CH₂-CH₂-CH=CH-,
-CH₂-C≡C-CH₂-CH₂-CH₂-CH₂-CH₂-, and
-CH₂-CH₂-CH₂-CH₂-CH₂-CH₂-CH(CH₃) -CH₂-.

Preferred Rₐ is a hydrocarbon residue containing 1-10 carbon atoms, preferably 1-8 carbon atoms. Rₐ may contain one or two side chains containing 1 carbon atom.

The configuration of the ring and the α- and/or ω chains in the above formula (I) and (II) may be the same as or different from that of the naturally occurring PGs. However, the present invention also includes a mixture of a compound having a naturally occurring type configuration and a compound of a non-natural type configuration.

In the present invention, any of isomers such as the individual tautomeric isomers, the mixture thereof, or optical isomers, the mixture thereof, a racemic mixture, and other steric isomers may be used in the same purpose.

The fatty acid derivative of the present invention is useful as a pharmaceutical composition for immunosuppression, and further as a pharmaceutical composition for treating inflammatory diseases, allergic diseases, and autoimmune diseases.

The term "treatment" and/or "improvement" used herein includes any means of control such as prevention, care, relief of the condition, attenuation of the condition and arrest of progression.

Examples of inflammatory diseases subject to the treatment in the present invention includes arthritis such as rheumatoid arthritis, pneumonitis, hepatitis including viral hepatitis, inflammation associated with infectious diseases, inflammatory bowel diseases, nephritis such as glomerulonephritis, gastritis, vasculitis, pancreatitis, peritonitis, bronchitis, myocarditis, encephalitis, psoriasis, dermatitis such as contact dermatitis, and the like. Further, in the present invention, the treatment of inflammatory diseases includes treatment of symptoms associated with inflammatory diseases such alopecia associated with dermatitis (alopecia pityroides, seborrheic alopecia, scarring alopecia, etc.).

Examples of allergic diseases subject to the treatment in the present invention include allergic conjunctivitis, allergic rhinitis, bronchial asthma, atopic dermatitis, food allergy, hay fever, and the like. Further, in the present invention, the treatment of allergic diseases include treatment of symptoms associated with allergic diseases such as the treatment of alopecia associated with atopic dermatitis.

Examples of autoimmune diseases subject to the treatment in the present invention include systemic lupus erythematosus, Sjogren's syndrome, multiple sclerosis, rheumatoid arthritis, pemphigoid, scleroderma, ulcerative colitis and the like. Further, in the present invention, the treatment of autoimmune diseases include the treatment of symptoms associated with autoimmune diseases.

The dose of the fatty acid derivative used in the present invention may be selected appropriately depending on the compound to be used, the strain of the subject, age, body weight, symptom to be treated, desired therapeutic effect, administration volume, term of treatment and the like. In general, a satisfactory effect can be obtained by the fatty acid derivative of the present invention in an amount of about 0.001 µg/kg - about 500 mg/kg per day, preferably about 0.01 µg/kg - about 50 mg/kg by systemic or topical administration of 1-4 times per day or continuous administration.

According to the present invention, the fatty acid derivative may be administered topically or systemically. Typically, the fatty acid derivative may be administered topically, orally, intranasally, by buccal administration, or by inhalation. It is preferred in the present invention that the fatty acid derivative is formulated as a pharmaceutical composition suitable for administration by a conventional method. The composition may be those suitable for topical, oral, intranasal, or buccal administration, administration by inhalation, intravenous administration (including instillation), perfusion or subcutaneous injection, and may be external agents, suppository, and pessary suitable for rectal administration, vaginal administration, and transdermal administration.

The pharmaceutical composition of the present invention may further comprise physiologically acceptable additives. The additives include components used with the compound of the present invention, such as excipients, diluents, fillers, solvents, lubricants, adjuvants, binders, disintegrating agents, coating agents, encapsulating agents, ointment bases, suppository base, aerosol agents, emulsifiers, dispersing agents, suspending agents, thickening agents, isotonic agents, buffering agents, soothing agents, preservatives, antioxidants, flavoring agents, aromatic agents, coloring agents, functional materials (such as cyclodextrins, biodegradable polymers), stabilizer and the like. These additives are well known to those skilled in the art and may be selected from those described in a conventional text of pharmaceutics.

The amount of the fatty acid derivative as defined above in the composition of the present invention may vary depending on its formulation and may be 0.001-10.0 w/v%, still more preferably 0.005-5.0 w/v%, most preferably 0.01-1.0 w/v%, in general.

A solid composition for oral administration of the present invention may include tablets, lozenges, sublingual tablets, capsules, pills, powders, granules and the like. The solid composition may be prepared by mixing one or more active ingredients and at least one inactive diluent. The composition further contain additives other than the inactive diluent, such as lubricants, disintegrating agents and stabilizers. If desired, tablets and pills may be coated with enteric film or gastrointestinal soluble film. The tablets and pills may be coated with two or more layers. In addition, the tablets and pills may be absorbed into a sustained release material, or may be microencapsulated. Furthermore, the present composition may be encapsulated using the readily degradable materials such as gelatin. The composition may be further dissolved in a suitable solvent such as a fatty acid or its mono-, di-, or triglycerides to prepare a soft capsule. It is also possible to use a sublingual tablet when immediate effect is required.

A liquid composition for oral administration may be emulsion, solution, suspension, syrup, and elixir. The liquid composition may further contain generally used inactive diluents such as distilled water and ethyl alcohol. Such composition may contain, in addition to the inactive diluent, additives such as adjuvant including lubricant and suspension, sweetening agent, flavoring agent, aromatic agents, preservatives, and the like.

The pharmaceutical composition of the present invention may be in the form of spraying composition comprising one or more active ingredients, which may be prepared according to a known method.

Examples of the intranasal preparations may be aqueous or oily solutions, suspensions or emulsions comprising one or more active ingredients. For the administration of an active ingredient by inhalation, the composition of the present invention may be in the form of suspension, solution or emulsion which can provide aerosol or in the form of powder suitable for dry powder inhalation. The composition for inhalational administration may further comprise a conventionally used propellant.

Examples of the injectable compositions of the present invention for parenteral administration include sterile aqueous or non-aqueous solutions, suspensions and emulsions. Diluents for the aqueous solution or suspension may include, for example, distilled water for injection, physiological saline and Ringer's solution.

Non-aqueous diluents for solution and suspension may include, for example, propylene glycol, polyethylene glycol, vegetable oils such as olive oil, alcohols such as ethanol and polysorbate. The composition may further comprise additives such as preservatives, wetting agents, emulsifying agents, dispersing agents and the like. They may be sterilized by filtration through, e.g. a bacteria-retaining filter, compounding with a sterilizer, or by means of gas or radioisotope irradiation sterilization. The injectable composition may also be provided as a sterilized powder composition to be dissolved in a sterilized solvent for injection before use.

Examples of the external agents include all the external preparations used in the fields of dermatology and otolaryngology, which includes ointment, cream, lotion, and spray.

Another form of the composition of the present invention is suppository or pessary, which may be prepared by mixing an active ingredient into a conventional base such as cacao butter that softens at body temperature, and nonionic surfactants having suitable softening temperatures may be used to improve absorbability.

The composition of the present invention may further comprise other ingredients as long as the object of the present invention is not impaired.

In the present invention, co-administration of the composition of the present invention and other agent(s) is also possible. Co-administration means that other agent(s) is administered prior to, simultaneously with (in one formulation or in a combination of different formulations), or after administration of the fatty acid derivative of the present invention. Agents which may be used in combination include, for example, steroids such as cortisol, prednisolone, triamcinolone, and dexamethasone, immunosuppressants such as cyclosporine, tacrolimus, and cyclophosphamide, non-steroidal antiphlogistic analgetics such as aspirin, indomethacin, and diclofenac.

Further details of the present invention will follow with reference to examples, which, however, are not intended to limit the present invention.

### EXAMPLES

### SYNTHESIS EXAMPLE 1

Isopropyl (5Z)-7-[(1R,2R,3R,5S)-2-[2-(2-heptyl-1,3-dioxan-2-ly)ethyl]-3,5-dihydroxycyclopentyl]hept-5-enoate (5)

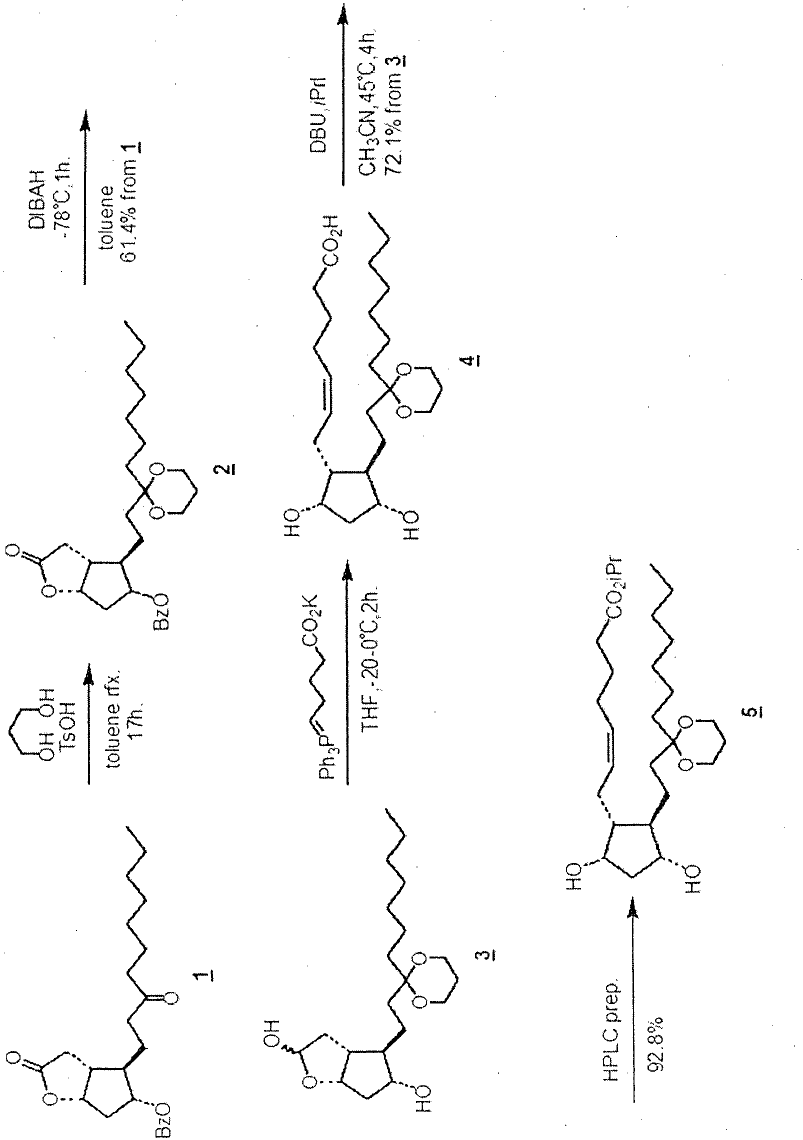

To the solution of compound 1 (510.0 mg, 1.273 mmol) in toluene (10.2 ml), 1,3-propanediol (0.92 ml, 12.73 mmol) and a catalytic amount of p-toluene sulfonic acid were added and the mixture was heated for 17 hours under reflux. After that, the reaction was left to stand so that it became room temperature, and washed with saturated aqueous sodium bicarbonate and saturated aqueous sodium chloride. The organic phase was dried with magnesium sulfate and evaporated under reduced pressure. The residue was purified by means of silica gel column chromatography (Merck 7734, Hexane: ethyl acetate=3:2) to give compound 2 (581.3mg).

The solution of compound 2 (580.0 mg, 1.265 mmol) in toluene (11.6 ml) was cooled to -78°C, 1.5M-DIBAH (in toluene, 2.95 ml, 4.427 mmol) was added dropwise thereto and the mixture was stirred for 1 hour, and then, methanol (1.79 ml) was added dropwise to the resulting mixture. Saturated aqueous Rochelle salt (100 ml) was added thereto and the mixture was vigorously stirred for 30 minutes. The resulting mixture was extracted with ethyl acetate, and the organic layer was washed with saturated salt water, dried with magnesium sulfate and evaporated under reduced pressure. The residue was purified by means of silica gel column chromatography (Merck 7734, Hexane: ethyl acetate=1:9-0:10) to give compound 3 (275.2 mg, yield 61.4% from 1).

To the dispersion of (4-carboxybuthyl)triphenyl phosphonium bromide (1.346 g, 3.038 mmol) in THF (6 ml), 1M- potassium t-butoxide in THF (6.07 ml, 6.07 mmol) at 0°C was added. The reaction was stirred for 1 hour at room temperature and then cooled to -20°C. Compound 3 (269.2 mg, 0.7594 mmol) in THF (7 ml) was added dropwise thereto and stirred for 2 hours at -20-0°C. Ice-cold water was added to the reaction, THF was removed by evaporation under reduced pressure. To the concentrated residue at 0°C, ice-cold 1N aqueous hydrochloric acid was added dropwise to adjust the solution to pH 4.

The solution was extracted with ethyl acetate and the organic layer was washed with saturated aqueous sodium chloride, dried with magnesium sulfate and evaporated under reduced pressure. The residue was added with ether and stirred for 17 hours at room temperature and then, filtrated with celite. The filtrate was evaporated under reduced pressure to give crude compound 4.

Compound 4 (0.7594 mmol) in acetonitrile (7.6 ml) was added with DBU (0.45 ml, 3.038 mmol), isopropyl iodide (0.30 ml, 3.038 mmol) and stirred for 4 hours at 45°C. The reaction mixture was evaporated under reduced pressure. The residue was added with water and extracted with ethyl acetate. The organic layer was washed with saturated aqueous sodium chloride solution, dried with magnesium sulfate and evaporated under reduced pressure. The residue was purified by means of silica gel column chromatography (Merck 9385, hexane : ethyl acetate= 2:3) to give 727.2 mg of the desired product (yield 72.1% from 3). Thus obtained compound 4 (carboxylic acid, 259.0 mg) was further purified by preparative HPLC to give compound 5 (isopropyl ester, 240.3 mg, HPLC purification yield 92.8%).

¹H-NMR spectrum (200MHz,CDCl₃) of compound 5: δ5.57-5.14(2H, m), 5.01(1H, sept, J=6.2Hz), 4.17(1H, bs), 3.97(1H, bs), 4.00-3.78(4H, m), 2.76(1H, d, J=6.2Hz), 2.29(2H, t, J=7.5Hz), 2.44-2.06 (5H, m,), 1.88(2H, bt,), 1.93-1.18(22H, m), 1.23(6H, d, J=6.2Hz), 0.89(3H, t, J=6.8Hz)

### SYNTHESIS EXAMPLE 2

Isopropyl (5Z)-7-[(1R,2R,3R,5S)-2-(3,3-dimethoxydecyl)-3,5-dihydroxycyclopentyl]hept-5-enoate (10)

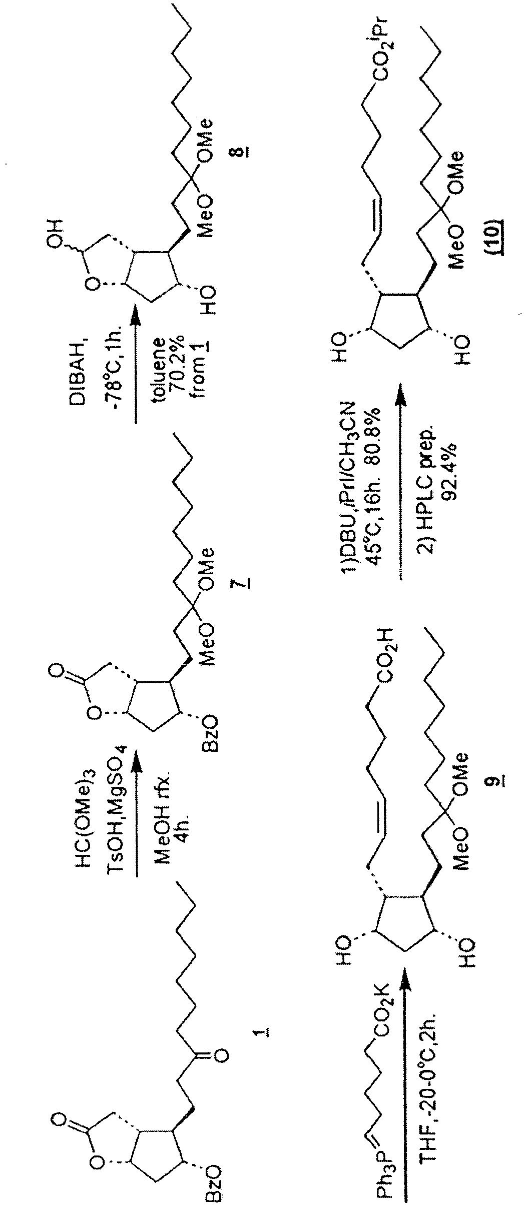

To the solution of compound 1 (797.8 mg, 2.002 mmol) in methanol (2.4 ml), a catalytic amount of p-toluene sulfate, methyl orthoformate(2.19 ml, 20.02. mmol) and unhydrous magnesium sulfate (1.20 g, 10.01 mmol) were added and heated under reflux for 4 hours. The reaction was cooled and added with sodium hydrogen carbonate, and filtered with celite. The filtrate was evaporated under reduced pressure and the residue was purified by means of silica gel column chromatography (Merck 7734g, hexane : ethyl acetate = 3:2) to give compound 7 (884.3 mg, yield 98.9%).

The solution of compound 7 (767.5 mg, 1.719 mmol) in toluene(15.4 ml) was cooled to -78°C, 1.5M-DIBAH (in toluene, 4.0 ml, 6.016 mmol) was added dropwise thereto and the mixture was stirred for 1 hour. Then, methanol was added dropwise to the reaction and the reaction was heated to room temperature. Saturated aqueous Rochelle salt (150 ml) was added thereto and the mixture was vigorously stirred for 30 minutes. The resulting mixture was extracted with ethyl acetate, and the organic layer was washed with saturated salt water, dried with magnesium sulfate and evaporated under reduced pressure. The residue was purified by means of silica gel column chromatography (Merck 9385, hexane: ethyl acetate=1:9) to give compound 8 (415.8 mg, yield 70.2%).

To the dispersion of (4-carboxybuthyl)triphenyl phosphonium bromide (1.250 g, 2.819 mmol) in THF, 1M-potassium t-butoxide in THF (5.64 ml, 5.64 mmol) at 0°C was added. The reaction was stirred for 1 hour at room temperature and then cooled to -20°C. Compound 8 (242.8 mg, 0.7048 mmol) in THF (4 ml) was added dropwise thereto and stirred for 2 hours at -20-0°C. Ice-cold water was added to the reaction, and THF was removed by evaporation under reduced pressure. To the residue at 0°C, ice-cold 1N aqueous hydrochloric acid was added dropwise to adjust the solution to pH 5. The solution was extracted with ethyl acetate and the organic layer was washed with saturated aqueous sodium chloride, dried with magnesium sulfate and evaporated under reduced pressure. The residue was added with ether and stirred for 17 hours at room temperature and then, filtrated with celite. The filtrate was evaporated under reduced pressure to give crude compound 9 (carboxylic acid).

To the solution of compound 9 (0.7048 mmol) in acetonitrile (7 ml), DBU (0.42 ml, 2.819 mmol), isopropyl iodide (0.28 ml, 2.819 mmol) were added and the mixture was stirred for 16 hours at 45°C. The reaction mixture was evaporated under reduced pressure. The residue was added with water and extracted with ethyl acetate. The organic layer was washed with saturated aqueous sodium chloride, dried with magnesium sulfate and evaporated under reduced pressure. The residue was purified by means of silica gel column (Merck 9385, hexane:ethyl acetate= 1:2) to give compound 10 (268.0 mg, yield 80.8% from 8).

Compound 10 obtained as above (total 370 mg) was further purified by preparative HPLC to give purified compound 10 (341.9 mg, HPLC purification yield 92.4%).
¹H-NMR spectrum (200MHz,CDCl₃) of compound 10: δ5.54-5.13(2H, m), 5.00(1H, sept, J=6.2Hz), 4.18(1H, bs), 3.95(1H, bs), 3.16(6H, s), 2.66(1H, d, J=6.4Hz), 2.29(2H, t, J=7.3Hz), 2.48-2.06(5H, m), 1.89(2H, bt), 1.79-1.17(20H, m), 1.23(6H, d, J=6.2Hz), 0.89(3H, t, J=6.8Hz)

### SYNTHESIS EXAMPLE 3

### Isopropyl (5Z)-7-[(1R,2R,3R,5S)-3,5-dihydroxy-2-{2-[2-(2-phenylethyl)-1,3-dioxolan-2-ly]ethy}cyclopentyl]hept-5-enoate (12)

Compound 12 was prepared from compound 11 in a same manner as Synthesis example 1. 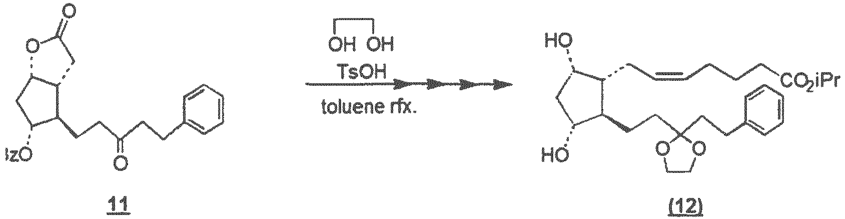 ¹H-NMR spectrum (200MHz, CDCl₃) of compound 11:δ8.04-7.93(2H, m), 7.63-7.38(3H, m), 7.35-7.11(5H, m), 5.21-5.03(2H, m), 2.98-2.24(11H, m), 2.12-1.98(1H, m), 1.80-1.50(2H, m)
¹H-NMR spectrum (200MHz, CDCl₃) of compound 12:δ7.35-7.12(5H,m), 5.56-5.35(2H, m), 5.00(1H, sept, J=6.2Hz), 4.15(1H, bs), 3.96(4H, s), 3.92(1H bs), 3.18(1H, bd), 2.86(1H, bd), 2.75-2.63(2H, m), 2.28(2H, t, J=7.3Hz), 2.46-1.15(17H, m), 1.22(6H, d, J=6.2Hz)

### SYNTHESIS EXAMPLE 4

### Ethyl(Z)-7-[(1R,2R,3R,5S)-2-(3,3-ethylenedioxydecyl)-3,5-dihydroxycyclopentyl] hept-5-enoate (15)

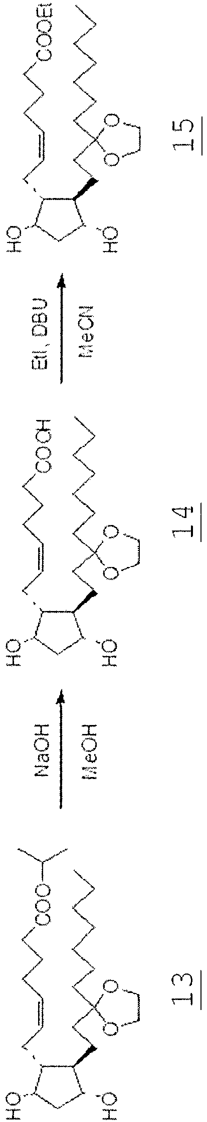

To the solution of compound 13 (9.18 g, 19.59 mmol) in methanol (91.8 ml), 8N-aqueous sodium hydroxide (24.49 ml) was added at 0°C. The reaction mixture was stirred for 3 hours at room temperature, and then acidified with 6N-hydrochloric acid at 0°C. The mixture was extracted with ethyl acetate (100 ml + 50 ml). The organic layer was washed with saturated aqueous sodium chloride (100 ml x 2), dried over anhydrous magnesium sulfate. The extract was evaporated under reduced pressure to obtain crude acid 14 as oil.

To the solution of crude acid 14 and 1,8-diazabicyclo[5.4.0]undec-7-ene (11.72 ml) in acetonitrile (60 ml), ethyl iodide (6.27 ml) was added dropwise at 0°C. The reaction mixture was stirred at 45°C for 17 hours, then cooled to room temperature, and evaporated. To the residue, water (100 ml) was added. The mixture was extracted with ethyl acetate (100 ml x 2). The organic layer was washed with O.1N-hydrochloric acid, saturated aqueous sodium bicarbonate (100 ml) and saturated aqueous sodium chloride (100 ml). The extract was dried over anhydrous magnesium sulfate and evaporated. The residue was purified by two times of silica gel column chromatography (Merck 7734, 220 g, hexane : ethyl acetate= 2:3, -> BW-300, 210 g, hexane:2-Propanol=6:1) to obtain ethyl ester 15 (8.60 g, 18.92 mmol, 96.6% from 13) as a colorless oil.
¹H-NMR (200MHz in CDCl₃ TMS = Oppm) of the compound 15: δ 5.58-5.29(2H, m), 4.15(1H, brs), 4.13(2H, q, J=7.1Hz), 3.97(1H, brs), 3.94(4H, s), 2.80-2.70(1H, br), 2.49-2.36(1H, m), 2.32(2H, t, J=7.4Hz), 2.36-2.15(4H, m), 1.90-1.83(2H, m), 1,83-1.12(20H, m), 1.26(3H, t, J=7.1Hz), 0.88(3H, t, J=6.5Hz).

### TEST EXAMPLE 1 (Immunosuppressive Effect)

### I. B Cell Proliferation Suppression Test

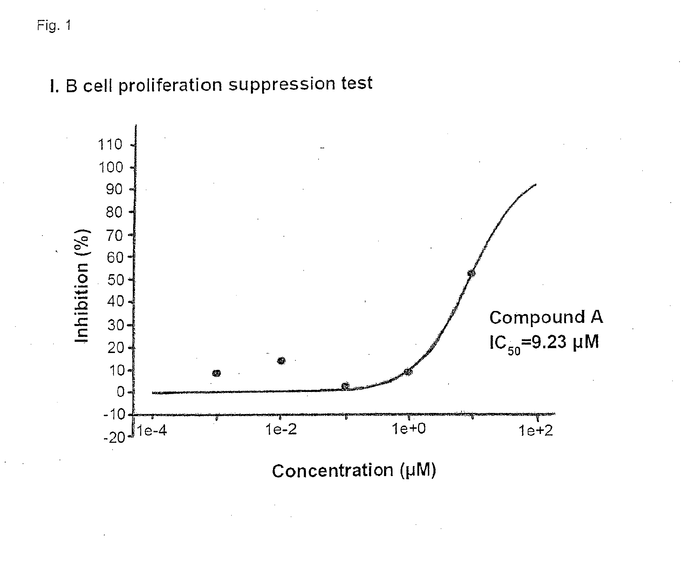

B lymphocytic cells (2×10⁶/mL) isolated from the spleen of Balb/c mice (weighing 17±1 g) were cultured in a pH 7.4 AIM-V medium containing a given concentration of compound A and 10 mg/mL of a lipopolysaccharide (LPS) at 37°C for 24 hours, and, after added with 120 nM ³H thymidine, further cultured for 16 hours. The cells were collected by filter filtration, and the uptake of thymidine into the cells was measured using a liquid scintillation counter to evaluate the cell proliferation-suppressing effect and calculate IC₅₀. The results are shown in Table 1 and Figure 1.

### II. T Cell Proliferation Suppression Test

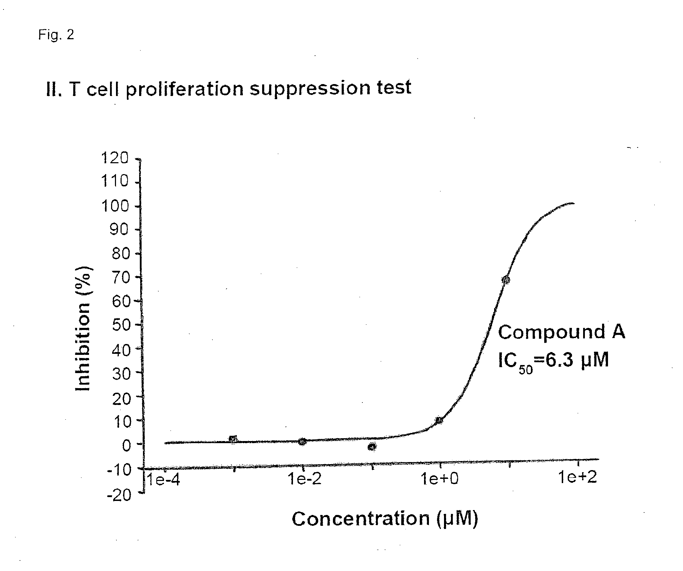

T lymphocytic cells (5×10⁶/mL) isolated from the thymus of Balb/c mice (weighing 17±1 g) were cultured in a pH 7.4 AIM-V medium containing a given concentration of compound A and 3.3 mg/mL of concanavalin A (Con A) at 37°C for 24 hours, and, after added with 120 nM ³H thymidine, further cultured for 16 hours. The cells were collected by filter filtration, and the uptake of thymidine into the cells was measured using a liquid scintillation counter to evaluate the cell proliferation-suppressing effect and calculate IC₅₀. The results are shown in Table 1 and Figure 2.

### III. NF-AT Gene Transcription Suppression Test

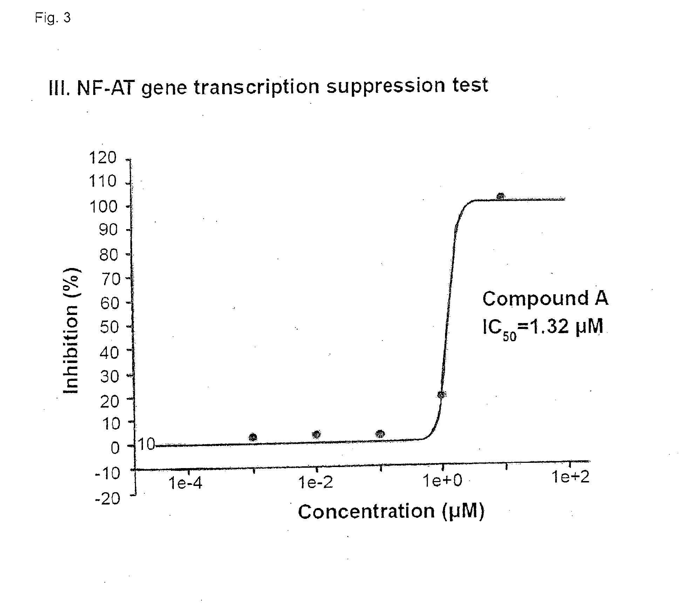

Human T lymphocyte Jurkat cells transformed with a β-galactosidase gene (lac Z) having a DNA binding site for the transcription factor NFAT-1 in its transcriptional control region (2.3×10⁶/mL) were cultured together with a given concentration of compound A in a pH 7.4 RPMI-1640 medium at 37°C for 20 minutes, and, after added with 0.5 mM A23187 (calcium ionophore) and 50 ng/mL PMA (Phorbol 12-myristate 13-acetate), further cultured for 4 hours. The activity of β-galactosidase was measured by conversion from FDG (fluorescein-di-beta-D-galactopyranoside) to fluorescein. Fluorescent emission was measured using a SpectroFluor Plus plate reader and IC₅₀ was calculated. The results are shown in Table 1 and Figure 3.

### IV. NF-κB Gene Transcription Suppression Test

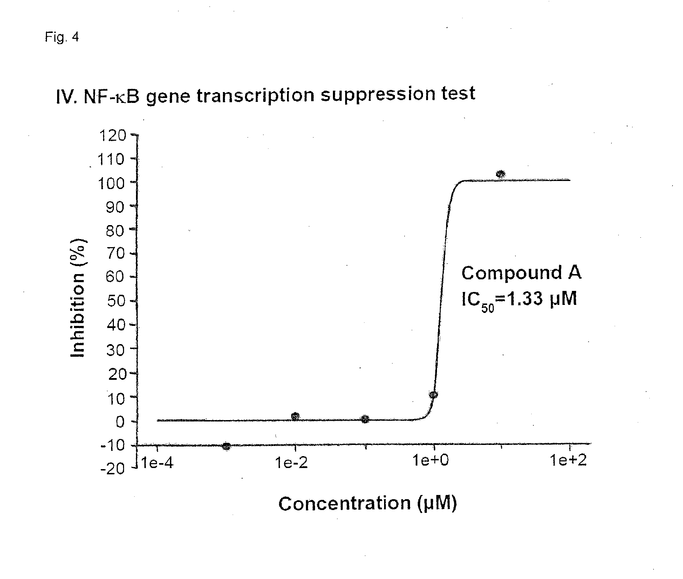

Human T lymphocyte Jurkat cells transformed with a β-galactosidase gene (lac Z) having a DNA binding site for the transcription factor NF-κB in its transcriptional control region (2.3×10⁶/mL) were cultured together with a given concentration of compound A in a pH 7.4 RPMI-1640 medium at 37°C for 20 minutes, and, after added with 0.5 mM A23187 (calcium ionophore) and 50 ng/mL PMA (Phorbol 12-myristate 13-acetate), further cultured for 4 hours. The activity of β-galactosidase was measured by conversion from FDG (fluorescein-di-beta-D-galactopyranoside) to fluorescein. Fluorescent emission was measured using a SpectroFluor Plus plate reader and IC₅₀ was calculated. The results are shown in Table 1 and Figure 4.

**[Table 1]**

| Test Name | Outline of Test Method | IC₅₀ for Compound A | Document for Test Method |
|---|---|---|---|
| B Cell proliferation suppression | Suppression of LPS-stimulated mouse B cell proliferation by the agent. | 9.23 µM | 1), 2) |
| T Cell proliferation suppression | Suppression of Con A-stimulated mouse T cell proliferation by the agent. | 6.3 µm | 1), 2) |
| NF-AT gene transcription suppression | Suppression of lac-Z gene transcription by the agent in human T Cells transformed with lac-Z gene. The transcription of lac-Z gene is controlled by the binding site to NFAT-1 transcription factor. | 1.32 µM | 3), 4) |
| NF-κB gene transcription suppression | Suppression of lac-Z gene transcription by the agent in human T Cells transformed with lac-Z gene. The transcription of lac-Z gene is controlled by the binding site to NF-κB transcription factor. | 1.33 µM | 5) |

### Compound A: isopropyl (Z)-7-[(1R,2R,3R,5S)-2-(3,3-ethylenedioxydecyl)-3,5-dihydroxycyclopentyl]hept-5-enoate Documents

1) Dayton JS, Turka LA, Thompson CB and Mitchell BS (1992) Comparison of the effects of myoribine with those of zothiaprine, 6 mercaptopurine and mycophenolic acid on T-lymphocyte proliferation and purine ribonucleotide metabolism. Mol Pharmacol. 1992;41:671-676.
2) Mishell BB, Shiigi SM and Eds (1980) Cell Proliferation in Selected Methods in Cellular Immunology, V, XXIX, W.H. Freeman Co., San Francisco, CA. pp153-160.
3) Emmel EA, Verweij CL, Durand DB, Higgins KM, Lacy E and Crabtree GR (1989) Cyclosporin A specifically inhibits function of the nuclear proteins involved in T cell activation. Science. 246:1617-1620.
4) Karttunen J and Shastri N (1991) Measurement of ligand-induced activation in single viable T cells using the lacZ reporter gene. Proc Natl Acad Sci USA. 88:3972.
5) Lenardo MJ and Baltimaore D (1989) NF-kB: a pleiotropic mediator of inducible and tissue specific gene control. Cell. 58: 227-229.
   (All of the above documents are incorporated herein by reference.)

### TEST EXAMPLE 2 (Test Using Atopic Dermatitis Mouse Model)

An atopic dermatitis mouse model (NC/NgaTndCrlj, male) induced by picryl chloride (PiCl) was used to evaluate effects against dermal inflammation and hair conditions on the skin suffering from dermal inflammation.

The test groups used were groups as shown in Table 2. PiCl-induced atopic dermatitis model mice were prepared by applying a PiCl solution to the sheared abdomen and footpad for sensitization and applying the PiCl solution to the sheared back and the right and left ears (both the inside and outside thereof) once a week for 5 weeks starting from the 5th day after sensitization for repeated induction. In each group, administration was performed once a day for 28 days starting from the 18th day after PiCl sensitization. Compound A and 0.1% tacrolimus ointment were openly applied to a ca. 3 cm × ca. 5 cm region of the neck and back of the model animals. The animals of the 5th group (the group of combined administration of prednisolone and 0.6 w/v% compound A) were orally administered 1 mg/kg of prednisolone and then transdermally administered 0.6 w/v% compound A. The animals of the 6th group (the group of combined administration of 0.1% tacrolimus ointment and 0.6 w/v% compound A) were administered 0.1% tacrolimus ointment at or beyond 3.5 hours after administration of 0.6 w/v% Compound A.

**[Table 2]**

| Group | Substance Administered | Dose | Route of Administration | Number of Cases |
|---|---|---|---|---|
| 1 | Base for Compound A | 100 µl/site | Transdermal | 10 |
| 2 | 0.08 w/v% Compound A | 100 µl/site | Transdermal | 10 |
| 3 | 0.3 w/v% Compound A | 100 µl/site | Transdermal | 10 |
| 4 | 0.6 w/v% Compound A | 100 µl/site | Transdermal | 10 |
| 5 | 1 mg/kg Prednisolone + 0.6 w/v% Compound A | Prednisolone: 10 mL/kg | Prednisolone: Oral | 10 |
| | | Comound A: 100 µl/site | Compound A: Transdermal | |
| 6 | 0.1 % Tacrolimus Ointment + 0.6 w/v% Compound A | Tacrolimus Ointment: 100 mg/site | Tacrolimus Ointment: Transdermal | 10 |
| | | Compound A: 100 µl/site | Compound A: Transdermal | |

Compound A: the same compound as in the Test Example 1

Each evaluation used a dermatitis score and a hair restoration score. The scores were obtained by grading dermatitis and hair growth/hair restoration conditions at Day 1, 8, 15, 22, and 29 (the next day after final administration) of administration, according to the following criteria.

<Dermatitis Findings> The dermatitis score of each individual was expressed as the sum of grades (0: subclinical to 3: severe) of the following dermatitis findings (1) to (5).
(1) Itching behavior: Behavior was observed for 2 minutes to examine the behavior of scratching the induction site.
   0: Subclinical: No scratching of the induction site is noted.
   1: Mild: Two or more continuously scratching behaviors for a period not exceeding about 1 minute in total are noted.
   2: Moderate: Scratching behaviors for a period of more than about 1 minute but less than about 1.5 minutes in total are noted.
   3: Severe: Scratching behaviors for a period of more than about 1.5 minutes in total or continuous scratching behaviors for 2 minutes are noted.
(2) Flare/bleeding: Flare and bleeding symptoms in the induction site were observed.
   0: Subclinical: No flare or bleeding symptom is noted in the induction site.
   1: Mild: Topical flare and/or bleeding symptoms are noted in the induction site, but no bleeding accompanied by continuous chafing is noted.
   2: Moderate: Sporadical flare and/or bleeding symptoms are noted in the induction site, or topical flare and bleeding symptoms accompanied by continuous chafing are noted.
   3: Severe: Flare and/or bleeding symptoms are noted throughout the induction site, or widely expanding continuous chafings with flare and bleeding symptoms are noted.
(3) Edema: The edema of the auricle as the induction site was qualitatively observed.
   0: Subclinical: The left and right auricles do not thicken.
   1: Mild: Either of the left and right auricles slightly thickens.
   2: Moderate: Both auricles clearly thicken and are taut.
   3: Severe: Both auricles clearly thicken, and are taut and curved, and feel rigid when touched with a finger.
(4) Chafing/tissue deficit: Chafings and tissue deficit symptoms were observed in the induction site.
   0: Subclinical: No chafing or tissue deficit symptom is noted in the induction site.
   1: Mild: Topical non-continuous chafings are noted in the induction site, but no tissue deficit is noted.
   2: Moderate: Sporadical chafings or small-size continuous chafings are noted in the induction site, but no tissue deficit is noted.
   3: Severe: Chafings are noted throughout the induction site or widely expanding continuous chafings and tissue deficit are noted.
(5) Crust formation/drying: Crust formation and drying symptoms were observed in the induction site.
   0: Subclinical: No crust formation or drying symptom in the induction site is noted.
   1: Mild: Topical crust formation and/or drying symptoms are noted in the induction site, the skin in the induction site is slightly whitened, and slight ablation of the cuticle is noted.
   2: Moderate: Sporadical crust formation and/or drying symptoms are noted in the induction site or sporadical ablation of the cuticle is clearly noted in the induction site.
   3: Severe: Crust formation and/or drying symptoms are noted throughout the induction site or ablation of the cuticle is clearly noted throughout the induction site.

<Hair Growth/Hair Restoration Condition> A region received transdermal administration (a ca. 3 cm x ca. 5 cm region of the neck and back) of each individual was observed.
0: No hair growth or hair restoration is noted in the observation site.
1: Hair growth and hair restoration are noted in 0 < 25% of the observation site.
2: Hair growth and hair restoration are noted in 25 ≤ 75% of the observation site.
3: Hair growth and hair restoration are noted in 75 ≤ 100% of the observation site.

### 1. Evaluation of Effect against Dermal Inflammation

An effect against atopic dermal inflammation was evaluated using the dermatitis score at Day 8 of administration. The results are shown in Table 3.

**[Table 3]**

| Dermatitis score (average ± standard error) when compound A was transdermally administered to picl-induced atopic dermatitis model mouse once a day for 8 days | | | | |
|---|---|---|---|---|
| Group | Base for Compound A | 0.08w/v% Compound A | 0.3w/v% Compound A | 0.1% Tacrolimus Ointment + 0.6w/v% Compound A |
| Number of Animals | 10 | 10 | 10 | 10 |
| Days of Administration 1 | 3.7 ± 0.4 | 3.8 ± 0.4 | 3.3 ± 0.5 | 3.3 ± 0.4 |
| 8 | 4.9 ± 0.7 | 3.7 ± 0.6 | 3.5 ± 0.4 | 2.2 ± 0.7 ^{$$} |

| | | | | |
|---|---|---|---|---|
| Significantly different from the base ($$: P<0.01 by Wilcoxon's test). | | | | |

It was shown that the progression of early dermatitis had a tendency to be suppressed in the groups administered with compound A compared to the group administered with the base for compound A. It was also shown that the progression of early dermatitis was significantly suppressed in the group receiving the combination of compound A and tacrolimus ointment compared to the group administered with the base for compound A.

The above results show that the fatty acid derivative of the present invention has the possibility of suppressing the progression of dermatitis such as atopic dermatitis and that the combination with an immunosuppressant is expected to have a further effect against atopic dermatitis.

### 2. Evaluation of Hair Condition in Skin Suffering from Dermal Inflammation

Hair condition in the skin suffering from dermal inflammation was evaluated using hair restoration scores at Day 15, 22 and 29 of administration. The results are shown in Table 4.

**[Table 4]**

| Hair restoration score (average ± standard error) when compound A was transdermally administered to picl-induced atopic dermatitis model mouse once a day for 28 days | | | | | | |
|---|---|---|---|---|---|---|
| Group | Base for Compound A | | 0.08w/v% Compound A | | 0.3w/v% Compound A | |
| No. of Animals | 10 | | 10 | | 10 | |
| Days of Administration 1 | 0.0±0.0 | (3.7±0.4) | 0.0±0.0 | (3.8±0.4) | 0.0±0.0 | (3.3±0.5) |
| 15 | 2.5±0.2 | (5.7±0.6) | 2.2±0.1 | (6.2±0.7) | 2.1±0.2 | (5.5±0.5) |
| 22 | 1.9±0.2 | (8.4±0.7) | 1.4±0.2 | (8.0±0.5) | 1.6±0.2 | (7.5±0.7) |
| 29 | 1.5±0.2 | (8.7±0.8) | 1.5±0.2 | (9.0±0.7) | 1.9±0.2 | (8.1±0.5) |
| | | | | | | |

| Group | 0.6w/v% Compound A | | 1 mg/kg Prednisolone + 0.6w/v% Compound A | | 0.1%Tacrolimus Ointment + 0.6w/v% Compound A | |
|---|---|---|---|---|---|---|
| No. of Animals | 10 | | 10 | | 10 | |
| Days of Administration 1 | 0.0±0.0 | (3.6±0.3) | 0.0±0.0 | (3.3±0.3) | 0.0±0.0 | (3.3±0.4) |
| 15 | 1.8±0.2 | (6.0±0.7) | 1.8±0.1 | (5.8±0.6) | 2.2±0.1 | (2.5±0.6 ^{$$}) |
| 22 | 1.5±0.2 | (8.3±0.4) | 1.1±0.1 | (7.3±0.8) | 2.1±0.1 | (3.8±0.8 ^{$$}) |
| 29 | 2.0±0.0^{#} | (9.3±0.6) | 2.2±0.2^{##} | (8.5±0.8) | 2.9±0.1 ^{##} | (5.4±0.9 ^{$}) |

| | | | | | | |
|---|---|---|---|---|---|---|
| The inside of parentheses indicates dermatitis scores. Significantly different from the Day 22 of administration (#: P < 0.05, ##: P < 0.01 by Wilcoxon's test). Significantly different from the base ($: P<0.05, $$: P<0.01 by Wilcoxon's test). | | | | | | |

In the group administered with the base for compound A, the hair restoration score was decreased (hair loss advanced) and the dermatitis score was increased (dermatitis progressed) after Day 15 on Day 22 and 29 of administration. On the other hand, in the groups administered with the agent(s), an increase in the hair restoration score (an improvement in the hair loss) was observed at Day 29 of administration compared to that of Day 22 of administration although the increase in the dermatitis score was observed at Day 22 and 29 of administration. This effect was observed in a dose-dependent manner in the compound A administration groups and also recognized in the group receiving the combination of compound A and any of prednisolone and tacrolimus ointment.

The above results demonstrate that the fatty acid derivative of the present invention suppresses and improves progression of hair loss accompanied by aggravation of dermatitis symptoms and can be combined with a steroid or an immunosuppressant. Particularly, in alopecia associated with (atopic) dermatitis, for which the treatment of dermatitis per se is first required in general, the fatty acid derivative of the present invention can suppress and improve hair loss even under the condition that aggravation of dermatitis symptoms progress.

## Claims

1. A pharmaceutical composition for immunosuppression which comprises as an active ingredient a fatty acid derivative represented by formula (I): 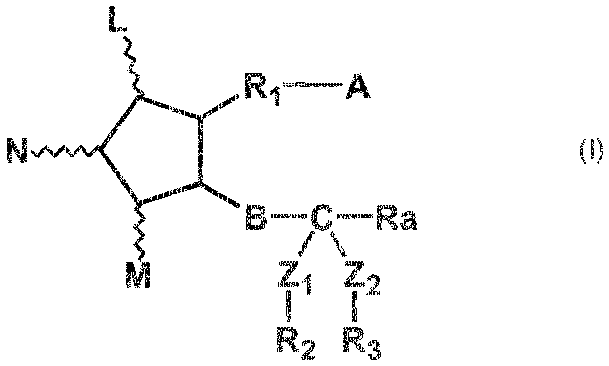
wherein L, M and N are hydrogen, hydroxy, halogen, lower alkyl, hydroxy(lower)alkyl, lower alkanoyloxy or oxo, wherein at least one of L and M is a group other than hydrogen, and the five-membered ring may have at least one double bond;
A is -CH₃, or -CH₂OH, -COCH₂OH, -COOH or a functional derivative thereof;
B is a single bond, -CH₂-CH₂-, -CH=CH-, -C≡C-, - CH₂-CH₂-CH₂-, -CH=CH-CH₂-, -CH₂-CH=CH-, -C≡C-CH₂- or -CH2-C≡C-;
Z₁ and Z₂ are oxygen, nitrogen or sulfur,
R₂ and R₃ are lower alkyl, or R₂ and R₃ are optionally linked together to form lower alkylene,
R₁ is a saturated or unsaturated bivalent lower or medium aliphatic hydrocarbon residue, which is unsubstituted or substituted with halogen, alkyl, hydroxy, oxo, aryl or heterocyclic group, and at least one of carbon atoms in the aliphatic hydrocarbon is optionally replaced by oxygen, nitrogen or sulfur; and
Ra is a saturated or unsaturated lower or medium aliphatic hydrocarbon residue, which is unsubstituted or substituted with halogen, oxo, hydroxy, lower alkyl, lower alkoxy, lower alkanoyloxy, cyclo(lower)alkyl, cyclo(lower)alkyloxy, aryl, aryloxy, heterocyclic group or hetrocyclic-oxy group; lower alkoxy; lower alkanoyloxy; cyclo(lower)alkyl; cyclo(lower)alkyloxy; aryl; aryloxy; heterocyclic group; heterocyclic-oxy group.

2. The pharmaceutical composition according to Claim 1, wherein L and M are hydroxy.

3. The pharmaceutical composition according to Claim 1 or 2, wherein A is -COOH or a functional derivative thereof.

4. The pharmaceutical composition according to any one of Claims 1-3, wherein B is -CH₂-CH₂-, -CH=CH-, or - C≡C-.

5. The pharmaceutical composition according to any one of Claims 1-4, wherein Z₁ and Z₂ are oxygen.

6. The pharmaceutical composition according to any one of Claims 1-5, wherein R₂ and R₃ are linked together to form lower alkylene,

7. The pharmaceutical composition according to Claim 6, wherein the lower alkylene is C3 alkylene.

8. The pharmaceutical composition according to any one of Claims 1-7, wherein the fatty acid derivative is isopropyl (Z)-7-[(1R,2R,3R,5S)-2-(3,3-ethylenedioxydecyl)-3,5-dihydroxycyclopentyl]hept-5-enoate.

9. The pharmaceutical composition according to any one of Claims 1-8 for the treatment of an inflammatory disease.

10. The pharmaceutical composition according to Claim 9 for treating skin inflammation or alopecia associated therewith.

11. The pharmaceutical composition according to Claim 10 for treating alopecia associated with skin inflammation.

12. The pharmaceutical composition according to any one of Claims 1-8 for the treatment of an allergic disease.

13. The pharmaceutical composition according to Claim 12 for treating atopic dermatitis or alopecia associated therewith.

14. The pharmaceutical composition according to Claim 13 for treating alopecia associated with atopic dermatitis.

15. The pharmaceutical composition according to any one of Claims 1-8 for the treatment of an autoimmune disease.

16. A method for immunosuppression in a mammalian subject, which comprises administering an effective amount of the fatty acid derivative of formula (I) as defined in Claim 1 to the subject in need thereof.

17. Use of the fatty acid derivative of formula (I) as defined in Claim 1 for immunosuppression in a mammalian subject.
